(19) **Europäisches Patentamt**
**European Patent Office**
**Office européen des brevets**

(11) **EP 2 378 971 B1**

(12) **EUROPEAN PATENT SPECIFICATION**

(45) Date of publication and mention
of the grant of the patent:
**20.02.2013 Bulletin 2013/08**

(51) Int Cl.:
*A61B 6/00* *(2006.01)*  *G06T 7/20* *(2006.01)*
*G06T 7/00* *(2006.01)*  *G10H 1/00* *(2006.01)*

(21) Application number: **09793594.4**

(22) Date of filing: **07.12.2009**

(86) International application number:
**PCT/IB2009/055533**

(87) International publication number:
**WO 2010/067293 (17.06.2010 Gazette 2010/24)**

(54) **FLOW SOUND IN X-RAY EXAMINATION**

STRÖMUNGSGERÄUSCH BEI RÖNTGENUNTERSUCHUNGEN

SON DE FLUX DANS UN EXAMEN RADIOLOGIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.12.2008 EP 08305926**

(43) Date of publication of application:
**26.10.2011 Bulletin 2011/43**

(73) Proprietor: **Koninklijke Philips Electronics N.V.**
**5621 BA Eindhoven (NL)**

(72) Inventors:
• **BONNEFOUS, Odile**
**NL-5656 AE Eindhoven (NL)**

• **FLORENT, Raoul**
**NL-5656 AE Eindhoven (NL)**
• **AUVRAY, Vincent, M. A.**
**NL-5656 AE Eindhoven (NL)**

(74) Representative: **Van Velzen, Maaike Mathilde**
**Philips**
**Intellectual Property & Standards**
**P.O. Box 220**
**5600 AE Eindhoven (NL)**

(56) References cited:
**WO-A1-96/31156    WO-A2-2008/107836**
**US-A- 4 729 379    US-A1- 2008 033 285**

Printed by Jouve, 75001 PARIS (FR)

**Description**

FIELD OF THE INVENTION

The invention relates to the representation of blood flow-related information. In particularly, the invention relates to an apparatus with an X-ray image acquisition device for representing blood flow-related information.

BACKGROUND OF THE INVENTION

Information concerning the blood flow is needed for different reasons. For example, vessel maps are commonly used for vessel analysis, neurological interventions or for the visualization of aneurysm structures. For example, information on the blood flow quality is needed before an intervention, such as a stent placement for instance, for planning purposes and after the intervention, e.g. the stenting, for an outcome control. But the recovery of satisfactory physiological blood flow cannot be verified. Usually, echography is used to get quantitative flow information. Indeed, Ultrasound is used for the exams of big vessels like carotid artery, abdominal aorta and lower limbs arteries. However, it is difficult to use ultrasound to image neurological vessels, due to the skull bone barrier. Difficulties using ultrasound also arise when examining coronary flows, due to the fast motion of the heart in front of the ultrasound probe and the rib cage barrier. From WO 2008/107836 a method is known to compute blood velocity using X-ray images of the vessel. US4729379A discloses the preamble of claim 1.

SUMMARY OF THE INVENTION

With contrast injection, X-ray produces dynamic sequences of flowing contrast agent within vessels. These images allow the detection and the localisation of vessel structures during intervention, useful for stent placement for instance. In the same way, it allows to get vessel maps for neurological intervention and visualization of aneurysm structures. Using contrast agents, X-ray is able to produce images of the anatomy of brain vasculature and coronary arteries during screening and interventional procedures. But it has shown that with common techniques in X-ray procedures, blood flow images are produced in such a way that the qualitative and quantitative information about the blood flow is difficult or even impossible to retrieve. For example, these images themselves do not provide the desired information for a detailed vessel analysis to the surgeon, for example. Even by combining several images to a sequence, for example representing a cardiac cycle, the derivable information is not sufficient. Hence, there may be a strong need for vascular functional information within an X-ray modality for cardiovascular exams and procedures that can be used for screening, but also for comparing the blood flow quality before and after an intervention. Thus, an object of the invention may be to provide an improved method in an X-ray imaging system for representing the blood flow, for vessel analysis purposes for example.

The object is reached with an examination apparatus according to the independent claim.

In an embodiment which doesn't form part of the invention, a method is provided for representing blood flow-related information in X-ray images comprising the following steps. First, a digital subtraction angiography DSA image is generated by generating a first image sequence, e.g. a pre-contrast image or mask image, with an X-ray imaging system, and by generating a second image sequence with the X-ray imaging system at a contrast phase; whereby in the second image sequence a part of the subject has a different contrast than in said first image sequence. For example, the contrast phase may include the introduction of a contrast medium into the subject to be examined. Further, a corresponding image of the first sequence to each image of the second sequence is determined, using a time registration process. Then, a first corresponding image is subtracted from second corresponding image generating the DSA image. A vessel registration is performed including the definition of a reference vessel image at a reference time after DSA automatically through the detection of sufficient contrast or by the user. Then, a registration between the reference vessel image and a current vessel image is performed and the current vessel image is warped towards the reference vessel image generating an output registered contrast image. Further, the output sequence is time filtered to extract time contrast modulation and vector velocity fields are computed applying motion estimation processing on the time filtered sequence. Finally, the velocity data is transformed into a sound signal.

According to an exemplary embodiment which doesn't form part of the invention, the image sequences are used to get vessel maps for neurological intervention and the visualization of aneurysm structure. Then, flow maps are produced using space time filtering and, for example, Optical Flow methods. These flow maps provide complex visual information about the actual flow characteristics. However, to comprehend this information, the user's visual attention is required. Knowing the spatial distribution of blood velocity, it is possible to artificially synthesize a sound defined by the visual spectrum determined by the velocity distribution. Providing the information to the acoustic sense, the user, e.g. the surgeon, is relieved in such a way that his visual attentiveness can be addressed to other aspects. Hence, providing a sound signal representing the blood flow will help clinicians to appreciate and qualify the vascular flow characteristics. It is also recognized that the sensibility of the ear is able to detect subtle variations, due to flow disturbances, or irregularities, more than any imaging representation. That means that by providing an acoustic representation of the blood flow, it is possible to provide more detailed information compared to a visual representation. Another advantage is that detailed blood flow information can be provided for regions of interest regardless of their situation within the body of patient, for example for neurological vessels or for obtaining information about coronary flows. Providing sound information based on X-ray imaging technique allows cardiologists and neurologists, for example, an enhanced way of evaluating vascular lesions and a better control on quality of resulting blood flow.

One of the advantages of the invention is that the operator, e.g. a physician in a clinic, is provided with information about the blood flow that is essential or at least very useful for example for further treatments or for analysis reasons. Due to the use of sound it is possible to provide detailed information such that said information can be perceived and used by the user in a very effective and timesaving way. This means that no additional devices such as an echography device are necessary for providing quantitative blood flow information when using an X-ray imaging device. This greatly enlarges the possible field of applications for X-ray imaging devices.

The vessel registration is necessary to differentiate global motion of vessels, which is caused by physiological motion, from flow motion within the vessel structures. The step of warping the current vessel image towards the reference image is using a registration process where the shape and the contours of the vessel structure are the elements considered to achieve the matching. The contrast variations within the vessel are then preserved. The registration and warping operations stabilize the artery and remove the global motion of the vessel, this latter being attached to a moving organ. After this step, the observed time/space variations of the contrast are due only to the motion of the contrast within the vessel and not to the moving vessel. With the registration and warping step it is possible to get rid of the motion in order to be in a better position to find the fluid motion. Fluid motion is then estimated in applying the time filtering. Computing vector velocity fields generates different aspects of blood flow information. Being able to measure directional local flow fields, it is then possible to derive any kind of parameter describing the flow characteristics. As a result the inventive method enables to extract a full flow field. Hence, the method produces a sound information representing the full flow field.

In an embodiment, which doesn't form part of the invention, the transforming of the velocity data into the sound data comprises the step of determining a local vessel orientation in a region of interest; the step of projecting the velocity vector on the vessel direction for each image corresponding to time $t$ of the velocity vector sequence; and the step of incrementing a histogram using the value of the velocity vector for each pixel.

In a further embodiment, which doesn't form part of the invention a method is provided wherein the sound signal reproduces characteristics of ultrasound Doppler examinations.

By adapting the sound characteristics to the sound impression of Ultrasound Doppler flow sound, a Doppler Spectrum analysis comparable to the known Ultrasound Doppler Flow Exam is provided. Indeed, Ultrasound Doppler exam is a commonly used reference exam for vascular evaluation. Hence, by providing a sound signal with the characteristics of an Ultrasound Doppler Flow sound, the clinician can use his skills from Ultrasound exams to interpret the sound and spectrum provided by the X-ray imaging system. Thus, the sound representation is beneficial for interpreting the X-ray results. By providing Doppler like sound information, the need for an interpretation of these new images by the user is avoided, which by the way would require huge clinical validations. In other words, reproducing results in a known manner, wherein the results are usually obtained with a different imaging modality, will enhance the acceptance and interpretation of X-ray base flow measure, because the clinician can appreciate the flow quality using the same skills he developed with ultrasound.

Contrary to Doppler ultrasound, where the sound is naturally produced by the Doppler Effect, i.e. the spectrum of the sound corresponds to the velocity distribution of blood flow, the sound signal according to the invention is synthetically generated. In Doppler ultrasound, high velocities give high-pitch sound, low velocities give low pitch tones, so that the Doppler sound varies from low to high pitch sound during the cardiac cycle, reproducing the pulsatility of the arterial flow.

In an exemplary embodiment, which doesn't form part of the invention, for the synthesized sound generating process a vessel direction map necessary for flow projection vector is computed using the vessel reference image. After a vessel segmentation, the vessel orientation is computed using standard oriented ridge filters. Then the vessel orientation is extended on the vessel map, creating the vessel direction map. This operation can be performed one time for the complete sequence. Then, each velocity frame is projected on the local vessel direction, producing the projected velocity frame.

Further, to synthesize the XR-flow sound, a region of interest (ROI) is determined by the user. For each projected velocity frame, the histogram of the flow field in the direction of the vessel axis is generated. This histogram is converted to an instantaneous pseudo spectrum with the adequate frequency scale. To do so, a maximum velocity can be chosen to correspond to a maximum frequency of the order of 2000 Hz. The synthesis is performed, for example, through the formula

$$SF(t) = \iint H(X, f, t)\cos(2\pi f t + \Phi(f, t))dxdf$$

stored in a memory, for example. The result is then read and converted in a voltage signal by a D/A. Finally, this signal is applied to a loudspeaker in order to provide the acoustic signal.

According to the invention, an X-ray image is provided in addition to the sound signal.

The user, i.e. the surgeon can thus be provided by a possibility to check and verify the information retrieved from the sound signal. Further, it is also possible for an assistant, for example, to (visually) control the blood flow during an operational step where loud noises cannot be avoided or where the sound signal is difficult to detect and thus difficult to interpret.

In a further embodiment, which doesn't form part of the invention, the registered contrast X-ray image is displayed on a display.

In a still further embodiment, which doesn't form part of the invention, the velocity data is transformed into a colour flow image and the colour flow image is superimposed to the X-ray image to generate a composite image, wherein the composite image is displayed on a display.

For example, the colour flow image is superimposed to the reference vessel X-ray image taken frozen at the reference time.

This allows presenting additional information that is included in the X-ray image, which results in a very significant image with high information density. The effect of the superimposition is that coloured pixel representing certain flow characteristics appear within the X-ray image that is traditionally only in black and white, i.e. in greyscale. This superimposition of coloured pixels can be accomplished by a covering overlaying, i.e. an opaque colour application over the greyscale image or by a semitransparent overlaying, i.e. where the greyscale image is still visible to a certain degree. Hence, as an effect the greyscale image partly appears coloured at least in some pixels.

According to an embodiment not forming part of the invention, the colour flow image reproduces characteristics of ultrasound colour flow imaging, at least concerning colour coding and flow direction.

This proposed imaging solution follows the ultrasound imaging example because ultrasound colour flow imaging is a reference for physicians and a very popular visualization technique that is broadly used and relied upon. Emulating this visualization method permits gaining the acceptance of X-ray clinicians for this new imaging technique.

Usually ultrasound uses blue/red colour maps. Flow direction is referenced by the ultrasound beam orientation, because ultrasound is limited to the velocity component parallel to the ultrasound beam. Red pixels correspond to blood moving away from the probe and blue pixels correspond to blood moving closer to the probe (or the contrary, this choice being defined by the user). The coded velocity value is then the projection of the velocity vector on the ultrasound beam direction. The colour (red/blue) codes the flow direction and the brightness codes the amplitude of the velocity projection.

In theory, knowing the angle between the vessel axis and the ultrasound beam, it is possible to estimate the velocity value. This assumes however that velocity vectors are parallel to the vessel axis, which is not the case for disturbed flows created by stenoses, bifurcations, stents and the like. Moreover, reverse flows produced by these anatomical singularities are naturally displayed in the opposite colour, and then easily detected.

As an effect, a coloured image appears that is colour coded just like an ultrasound image. But this coloured image appears in an X-ray environment, where images are commonly greyscale, without the necessity of additional ultrasound devices. In case of a superimposition over an X-ray image, a combination of an X-ray image and an ultrasound colour-coded image appears. The effect is similar to the one described above, i.e. a greyscale image shows coloured pixels at least in certain areas, but the colours are applied according to the ultrasound colour code. This means that they represent the same type of information in the X-ray image.

According to an embodiment not forming part of the invention, a vessel direction map is created for the velocity data using the vessel reference image comprising the steps of vessel segmentation, computing vessel orientation using a standard oriented ridge filter and extending the vessel orientation on a vessel map.

This enables an improved consideration of the actual flow characteristics since the velocity vectors can be set into relation with the vessel direction.

In an embodiment not forming part of the invention, components of the velocity field being longitudinal in respect of the vessel axis are computed, a threshold for the blood flow velocity is determined producing artificial aliasing patterns and the longitudinal flow is transformed into sound data for the sound signal using the velocity threshold.

These artificial patterns mimic an intrinsic aliasing effect, which occurs for high velocities in Ultrasound CFI keeping a good colour dynamics for normal flows and enhancing the high flows that are typically associated to tight stenoses. To produce this aliasing phenomenon if velocity values are abnormally high, in one embodiment the threshold is corresponding to normal maximum blood flow velocities.

To give more detailed flow information, it is foreseen that components of the velocity field being transverse corresponding to a projection perpendicular to the vessel axis are computed and the transverse flow is transformed into sound data for the sound signal.

In a still further embodiment not forming part of the invention, flow variance and/or flow vorticity of the velocity field are computed and transformed into sound data for the sound signal. This provides the user with very detailed information about the actual blood flow that can be compared to the information density achievable with Ultrasound CFI.

In one embodiment not forming part of the invention, the vector velocity field is computed by the means of optical flow methods.

Detailed information to be evaluated in further processing steps can be achieved with optical flow methods, especially with multi-scale optical flow methods. Optical flow processing in particular produces flow sequences, which contain velocity maps in time and space without the necessity to rely on models such as flow models or geometrical models.

In a further embodiment not forming part of the invention, the contrast phase is submitted to the arterial pulsed pressure creating a periodic time modulation of the contrast density and a time filter response is tuned to the cardiac frequency of a subject to be examined such that the reference vessel image and/or the current vessel image are synchronized with the cardiac period of a subject to be examined.

It has shown that the contrast phase within an artery submitted to the arterial pulsed pressure creates a periodic time modulation of the contrast density. With the blood flow, this modulation is transported in the arterial network, creating a kind of "contrast wave" pattern. By tuning the characteristics of a dedicated time filter to the cardiac period it is possible to enhance the contrast wave pattern. This sequence is an attractive visualization of the flowing contrast and may be the input for the velocity vector field estimation step.

In a further embodiment not forming part of the invention, components of the velocity field that are transverse corresponding to a projection perpendicular to the vessel axis are computed and the transverse flow is transformed into sound data for the sound signal.

According to the invention, the object is achieved with an examination apparatus for representing blood flow-related information that comprises at least one X-ray image acquisition device, a data processing unit and at least one sound device. The X-ray image acquisition unit is arranged to generate a first image sequence and to generate a second image sequence at a contrast phase, whereby in the second image sequence a part of the subject has a different contrast than in said first image sequence. The data processing unit is arranged to determine a corresponding image of the first sequence to each image of the second sequence, using a time registration process; to subtract the first corresponding image from the second corresponding image to generate a DSA image; and to register the vessel including a definition of a reference vessel image at a reference time after DSA through the detection of sufficient contrast or by the user; to register between the reference vessel image and a current vessel image; and to warp the current vessel image towards the reference vessel image generating an output registered contrast image; and to time filter the output sequence to extract time contrast modulation; and to compute vector velocity fields applying motion estimation processing on the time filtered sequence; and to transform the velocity data into sound data generating a sound signal. The at least one sound device is arranged to provide the sound signal.

In a further preferred embodiment the apparatus also comprises a display. The data processing unit is arranged to transform the velocity data into a colour flow image and the display is arranged for displaying the registered contrast X-ray image and the colour flow image superimposed to the X-ray image.

According to the invention, the object is also achieved with an X-ray imaging system with an apparatus according to one of the above-described embodiments.

According to a further exemplary embodiment not forming part of the present invention, a computer program element is provided that is characterized by being adapted to perform the steps of the method according to one of the preceding embodiments.

This computer program element might therefore be stored on a computing unit, which might also be part of an embodiment of the present invention. This computing unit may be adapted to perform or induce the performing of the steps of the method described above. Moreover, it may be adapted to operate the components of the above described-X-ray imaging system. The computing unit can be adapted to operate automatically and/or to execute the orders of a user.

This embodiment covers both a computer program, that right from the beginning uses the invention, and a computer program, that by means of an update turns an existing program into a program that uses the invention.

Further on, the computer program element might be able to provide all necessary steps to fulfil the procedure of representing blood flow-related information in X-ray images as described above.

According to a further embodiment not forming part of the present invention, a computer-readable medium is presented wherein the computer-readable medium has a computer program element stored on it which computer program element is described by the preceding section.

According to a further embodiment not forming part of the present invention, a medium for making a computer program element available for downloading is provided, which computer program element is arranged to perform the method according to one previously described embodiment of the invention.

An embodiment of the invention will be described hereinafter with reference to the figures. The above-mentioned aspects and other aspects will be apparent from this description.

BRIEF DESCRIPTION OF THE DRAWINGS

Fig. 1    schematically describes an X-ray imaging device according to the invention;
Fig. 2    schematically shows the general processing scheme;
Fig. 3    schematically presents operations corresponding to the flow sound synthesis procedure of figure 2;
Fig. 4    schematically presents further operations concerning the flow sound synthesis procedure of figure 2;
Fig. 5    schematically presents further operations concerning the flow sound synthesis procedure of figure 2;
Fig. 6    schematically shows the general processing scheme with an additional generation of a colour flow image;
Fig. 7    schematically presents the operations for generating the colour flow image;
Fig. 8    shows an example for a visualisation of a velocity spectrum; and
Fig. 9    shows an image of the visualisation of Fig. 8.

DETAILED DESCRIPTION OF EMBODIMENTS

In order to produce a flow sound, a first sequence of images is acquired by an X-ray image acquisition device. Further, a second sequence of images is acquired with injected contrast agent. Then, the contrast signal is extracted through a DSA operation. Further, the vessel structure is registered to cancel the motion of the vessels. Then, time filtering of the sequence is used to enhance the moving components of the contrast. The images are then used for generating vector velocity fields which are represented by contrast motion within the vessels. This extraction is achieved with an optical flow (OF) method. The output of the OF operation is then used to produce velocity distribution curves. This graphical information is then converted into a synthetic sound signal.

In a preferred embodiment, the sound signal is adapted to the acoustic Doppler effect.

Hence, an acoustic signal is generated that mimicks the acoustic effect that is used during Ultrasound examinations. In Doppler Ultrasound systems the user points the vessel location on the screen, and an ultrasound beam transmits ultrasound pulses on the artery. The flowing blood reflects a signal, yielding a Doppler Effect due to the flowing blood. In case of a moving object, such as blood flowing towards to or away from the ultrasound source, the reflected sound detected by the receiver is showing a slight alteration in its frequency and wavelength. For example, compared to the emitted frequency the frequency received is increased when an object is moving towards the sound source. When the object, e.g. the blood is moving away, the received frequency is decreased compared to the emitted frequency. After a simple baseband demodulation of the reflected signal, the frequency modulation represents the velocity distribution of flow. The sound spectrum being in the range of the human ear spectrum, this sound is used to judge the blood flow characteristics.

According to the invention, a sort of sound basis is generated that sounds like the Doppler sound produced in Ultrasound examination. The graphically detected blood flow information is used to shape the spectrogram of the sound signal as if the detected blood motion was producing a Doppler signal through an Ultrasound exam. In other words, the detailed information about the blood flow characteristics is presented as an acoustic information to the user in the same manner as if an Ultrasound device was used.

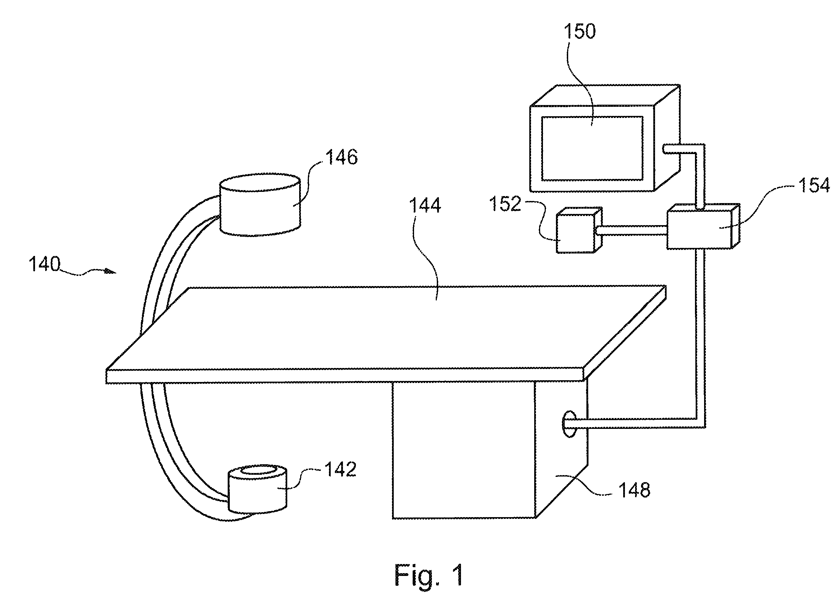

Fig. 1 schematically shows an X-ray imaging system 140 for representing blood flow-related information. Note that the example shown is a so-called C-type system. Nevertheless, the invention also relates to other types of X-ray imaging systems. A source of X-ray radiation 142 is provided to generate X-ray radiation. Further, a table 144 is provided to receive a subject to be examined, and an X-ray image detection module 146 is located opposite the source of X-ray radiation 142, i.e. during the radiation procedure, the subject is located between the source of X-ray radiation 142 and the detection module 146. The latter is sending image data to a data processing unit 148, which is connected to both the detection module 146 and the source 142. Furthermore a display 150 is arranged in the vicinity of the table 144 to display information to the person operating the X-ray imaging system, i.e. a clinician. Preferably the display 150 is movably mounted in order for an individual adjustment depending on the examination situation. Further, a sound output 152 device is provided, for example in form of at least one loudspeaker. The sound output 152, i.e. the loudspeaker can, for example, be integrated into the display device 150. Also, an interface unit 154 is arranged to input information by the user.

Basically the image detection module 146 generates X-ray images that are further processed in the data processing unit 148, said procedure being described more detailed in the following. As a result of the process in a preferred embodiment of the invention a sound signal or sound information representing the blood flow information is provided by the sound output device 152. Additionally, an X-ray image based image is displayed on the display 150 to the clinician. As clinicians are usually trained to detect sound images concerning blood flow when using Ultrasound examination, in an exemplary embodiment the sound information provided is mimicking the acoustic Doppler effect used in Ultrasound examination.

To provide an enhanced detection of the full flow information, the image shown in addition is adapted to the colour coding used in ultrasound colour flow imaging. In other words, the colour flow image reproduces the characteristics of ultrasound colour flow imaging, at least concerning colour coding and flow direction. Therefore, the image displayed on the display 150 is a colour image superimposed to an X-ray image which is commonly in black and white, respectively in greyscale.

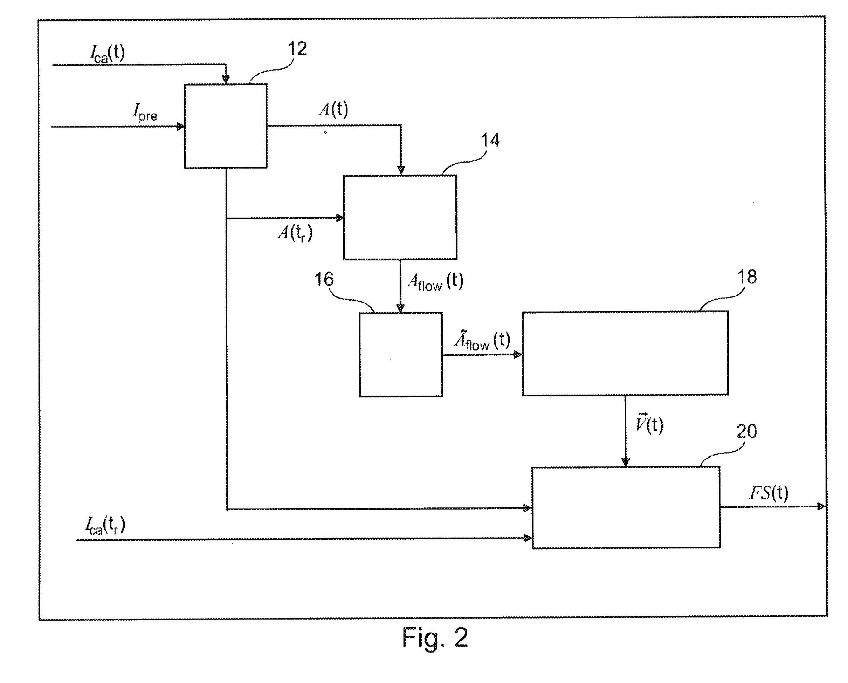

For generating the sound signal according to the invention, the following steps are provided. Figure 2 presents the general processing scheme, corresponding to these steps.

Prior to the actual processing steps, a DSA operation 12 is provided. This operation is necessary to extract a contrast signal. As an input data, two image sequences are provided by an X-ray image acquisition device: a first sequence $I_{pre}(t)$ is acquired before a contrast phase, i.e. for example a contrast injection. Then a contrast agent may be injected or introduced in a suitable manner into the vessel to be examined. A second sequence $I_{ca}(t)$ is acquired during the contrast injection. For each image of the second sequence $I_{ca}(t)$, a corresponding image of the first sequence $I_{pre}(t)$ is determined, using an adequate time registration process. Then, the subtraction is performed between these two images, producing a DSA image $A(t)$, which contains only vessels filled with contrast agent, i.e. the contrasted image of the vessel.

Further, vessel structures are registered to cancel the motion of the vessels. This means that it is necessary to differentiate global motion of the vessels, due to physiological motion, from flow motion within the vessel structures. Therefore a vessel registration 14 is performed between a reference vessel image $A(t_r)$ chosen after the DSA procedure and a current vessel image $A(t)$. the reference image. For example, the reference image $A(t_r)$ is chosen by the user. In a preferred embodiment the reference image is automatically chosen through the detection of sufficient contrast filling.

The current vessel image $A(t)$ is then warped towards $A(t_r)$, using a registration process where the shape and the contours of the vessel structure are the elements considered to achieve the matching. Contrast variations within the vessel structure are then preserved. The output registered contrast image is called $A_{flow}(t)$.

Further, the output sequence is time filtered to extract time t contrast modulation. It is noted that the motion estimation involved performing these registration and warping operations concerns the tube motion. The inventive method results in the possibility to differentiate vessel motion from fluid motion. With respect to registration and warping, several approaches for a transformation of coordinates may be envisaged like block matching or parametric motion estimation.

Then, in a preferred embodiment, a time filtering process 16 of the sequence is used to enhance the moving components of the contrast. It has shown that the contrast injection within an artery submitted to the arterial pulsed pressure creates a periodic time modulation of the contrast density. With the blood flow, this modulation is transported in the arterial network, creating a kind of "contrast wave" pattern. It is possible to tune the characteristics of a dedicated time filter to the cardiac period, and enhance the contrast wave pattern, creating the sequence $\tilde{A}_{flow}(t)$. This sequence is an attractive visualization of the flowing contrast and is an input for the next step, which is a contrast flow velocity vector field estimation step 18.

In the velocity vector field estimation step 18 velocity vector field representing contrast motion within vessels is extracted with an adequate Optical Flow (OF) method. The sequences $A_{flow}(t)$ or $\tilde{A}_{flow}(t)$ are used to compute the velocity vector field. Preferably, Multiscale Optical Flow (MOF) methods are implemented to estimate the vector flow field, producing the vector sequence V(X,t). The output of the OF operation is then used to create the colour velocity image. Even though other motion estimations are also imaginable to estimate velocities, such as block matching or local affine motion modelling, it has been found that optical flow can be specifically tailored to the problem of motion estimation. Multiscale optical flow allows to gradually increase the resolution of the flow estimation. The latter is a very important aspect to detect local vorticity of the flow for example (also, see below).

So far, the use of successive image processing steps described above allows the extraction of the blood velocity vector field. Finally, in order to provide this information to the user, an adequate synthesized sound generating step 20 is provided.

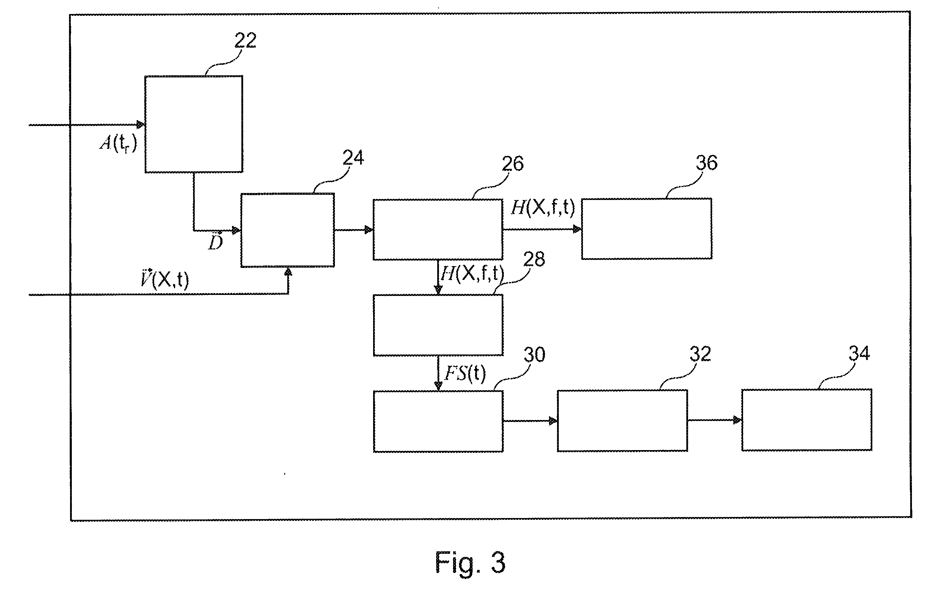

The synthesized sound generating 20 is schematically shown in Fig. 3 and described in more detail below. A vessel direction map $\vec{D}$ necessary for flow projection vector is computed in a vessel geometry determining step 22 using the vessel reference image $A(t_r)$. After a vessel segmentation, the vessel orientation is computed using standard oriented ridge filters. Then the vessel orientation is extended on the vessel map, creating the vessel direction map $\vec{D}$. This operation is performed one time for the complete sequence. Then, in a velocity projection step 24, each velocity frame $V(X,t)$ is projected on the local vessel direction D, producing the projected velocity frame $Vp(X,t)$.

To synthesize the XR-flow sound, a region of interest (ROI) is determined by the user. For each projected velocity frame $Vp(X,t)$, the histogram of the flow field in the direction of the vessel axis is generated in a velocity histogram generation 26. This histogram is converted to an instantaneous pseudo spectrum with the adequate frequency scale, $H(X, f,t)$ in a sound synthesis step 28. To do so, a maximum velocity is chosen to correspond to a maximum frequency of the order of 2000 Hz. The synthesis 28 is performed, for example, through the formula

$$SF(t) = \iint H(X, f, t) \cos(2\pi f t + \Phi(f, t)) dx df$$

stored in a memory 30. The result is then read and converted in a voltage signal by a D/A converting step 32. Finally, this signal is applied to a loudspeaker 34 in order to provide the acoustic signal. For example, the loudspeaker 34 can be part of the sound output device 152 described in Fig. 1.

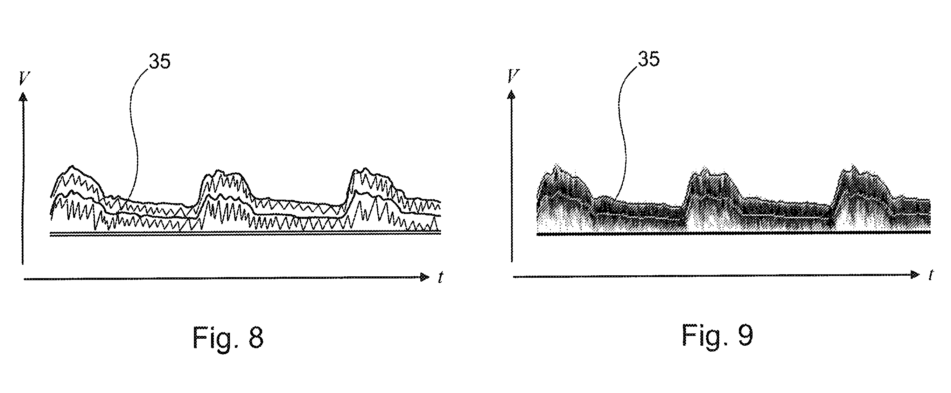

To provide enhanced information to the user, i.e. the surgeon, in one exemplary embodiment the (pseudo) spectrum of the blood stream is displayed in form of an instantaneous spectrogram, i.e. a velocity spectrum 35 is visualised in a visualisation step 36. It is noted that this visualisation 36 is an option that can be added. An example for the velocity spectrum 35 visualisation is shown in Fig. 8 in a graphical manner and in Fig. 9 as an image of the same velocity spectrum 35.

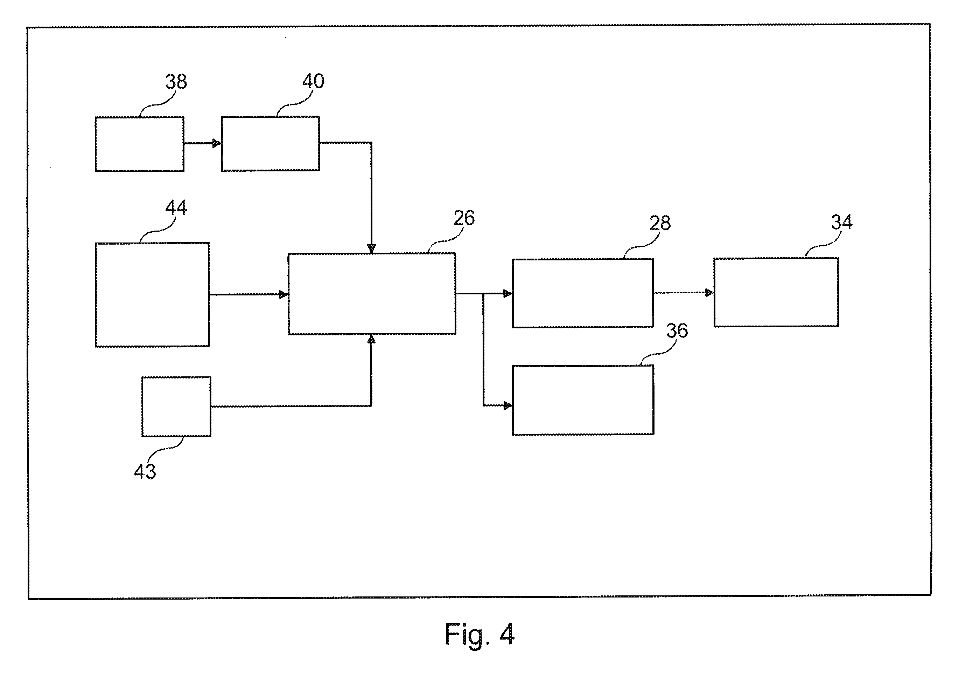

Fig. 4 shows the principle of the velocity histogram production 26 and its use. Using a mask 38 of the vessel obtained by adequate thresholding of the X-ray image, the local vessel orientation 40 is determined in a region of interest (ROI) 42, defined by the user for example.

For each image corresponding to time $t$ of the velocity vector sequence 44, for each pixel in the ROI 42, the velocity vector is projected on the vessel direction. Its value $V$ allows to increment a histogram value for the velocity histogram 26.

In this way, after considering all pixels of the ROI 42, the velocity distribution is computed and represented by the velocity histogram $H(v,t)$ 26. This velocity histogram 26 may be visualized in the same way as, for example, Doppler Spectrum of ultrasound systems. Indices (resistance, pulsatility etc.) characterising the flow curves may be computed in the same way as in an Ultrasound Doppler system, using an image of the histogram 26.

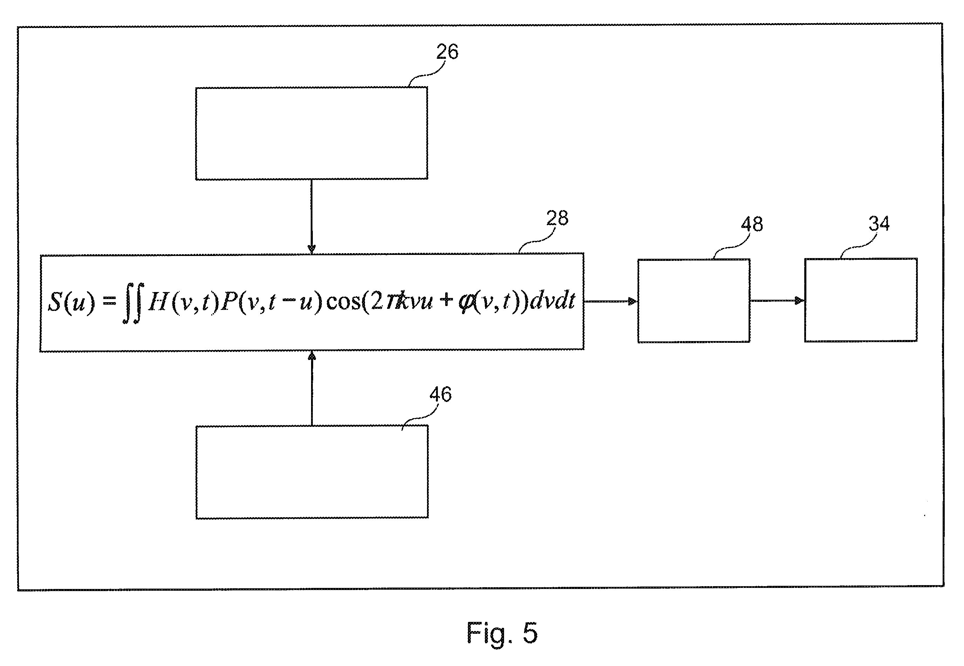

Figure 5 shows the principle of the sound synthesis 28 for an embodiment where the transformed sound signal is adapted to the sound of an Ultrasound Doppler examination apparatus. The histogram function $H(v,t)$ 26 is the basis of the Doppler sound synthesis. The velocity parameter is transformed into a sound frequency parameter in the synthesis process 28, thus reproducing the transformation of ultrasound signal due to the Doppler effect. $H(v,t)$ is here considered as a spectrogram. The complete distribution of the velocity (and not only the mean velocity) is used in the synthesis 28 in order to obtain the same sound complexity of true Doppler sound, where every particle crossing the ultrasound beam contributes to the sound tone.

Therefore, several parameters must be predetermined in an input step 46 to produce this sound. For example, they can be automatically set or they can be set by the user. Indeed, a frame rate of 15 to 300 Hz of the X-ray sequence $I_{pre}$ is low compared to the frequency of the audible sound, whereas current sound sampling frequency is in the order of about 10 kHz. This requires a strong interpolation. This is addressed by a parameter k called frequency multiplier, and a kernel or sound shape $P(v,t)$. This kernel allows also to apply dedicated frequency shape to the frequency-velocity distribution, in order to adapt the resulting sound to the ear response.

Further, the input step also includes a random phase distribution. Therefore, by a random phase function $ø(v, t)$. a coloured noise signal is created with the spectrogram H. This allows mimicking Doppler sound generated by particles crossing ultrasound beam. For example, the resulting sound may be obtained with the expression:

The synthesized sound is then converted 48 and finally provided as the sound signal.

However, according to an exemplary embodiment of the invention, an X-ray image is displayed on a display in addition to the sound signal. Since the sound is representing the blood flow, may it be mimicking the ultrasound Doppler effect or may it be in some other sound coding, the acoustic output is an essentially varying sound. As an effect, when both informative signals, i.e. the acoustic and the visual information, relate to the same parameters of the same ROI, the changes in the two signals will occur corresponding to each other. Simply said, a variation in the sound signal will have a parallel visual variation in the image displayed.

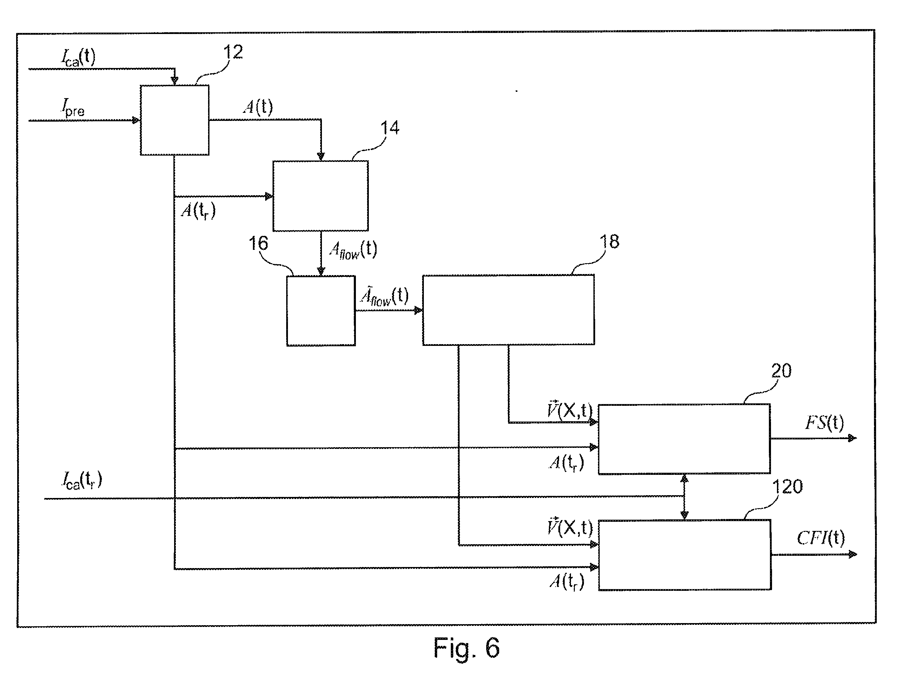

In a further embodiment shown in Fig. 6 the same flow data resulting from the velocity vector field estimation step 18 is also used for an adequate colour mapping transformation 120.

For a better understanding it may be beneficial to recall characteristics of ultrasound colour flow imaging (Ultrasound CFI). One aspect to be considered is the flow direction. Flow direction is referenced by the ultrasound beam orientation, because ultrasound is limited to the velocity component parallel to the ultrasound beam. Usually Ultrasound CFI uses Blue/Red colour maps. Red pixels correspond to blood moving away from the probe. Blue pixels correspond to blood moving closer to the probe. Of course this colour coding can be the contrary or altered somehow depending on the needs of the user.

The coded velocity value is the projection of the velocity vector on the ultrasound beam direction. The colour (red/blue) codes the flow direction and the brightness codes the amplitude of the velocity projection. In theory, knowing the angle between the vessel axis and the ultrasound beam, it is possible to estimate the velocity value. This assumes however that velocity vectors are parallel to the vessel axis, which is not the case for disturbed flows created by stenoses, bifurcations, stent and the like. Moreover, reverse flows produced by these anatomical singularities are naturally displayed in the opposite colour, and then easily detected.

Another aspect is that Ultrasound CFI is submitted to an intrinsic aliasing effect that occurs for high velocities. This aliasing allows keeping a good colour dynamics for normal flows and enhancing the high flows typically associated to tight stenoses. This natural feature is strongly used by physicians.

Further, disturbed flows may be of interest. Therefore other colour images are also produced by Ultrasound CFI. So-called flow variance indexes, measured by colour Doppler techniques, may be displayed. High variance corresponds to disturbed flows, produced by flow jets or other singularities. It may be associated to an aggressive interaction between flow and arterial wall. Usually, velocity variance is coded with a green colour. It may be also mixed to the velocity map, producing a red-yellowish and blue-greenish look up table to image velocity and velocity variance together.

For the X-ray CFI coding according to the invention, in the embodiment shown as an example only those characteristics of Ultrasound CFI are reproduced when they are of clinical advantage. Of course, in case there are limitations they can be transformed in a suitable manner.

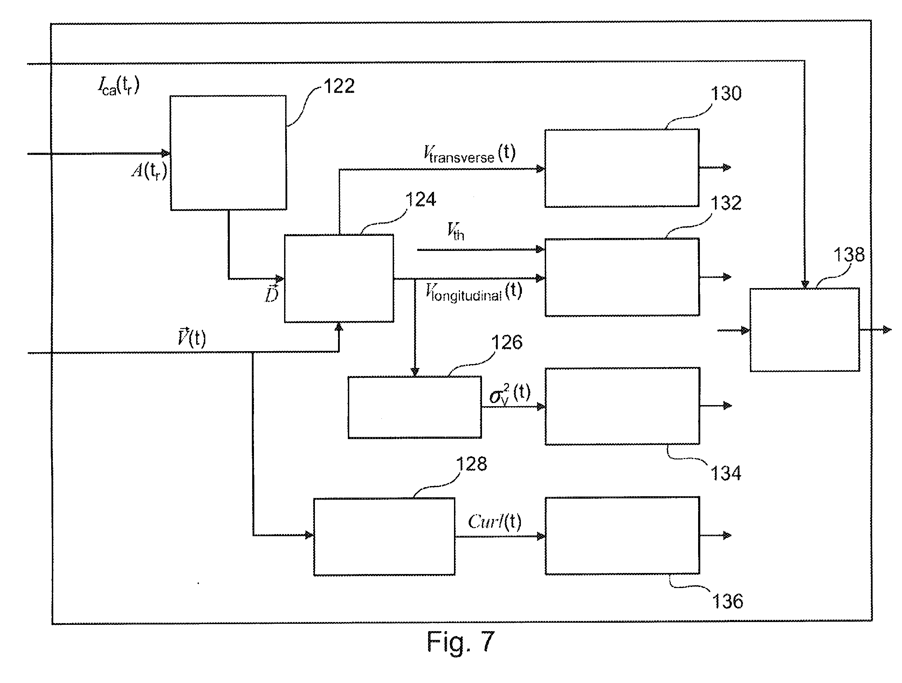

Hence, flow direction, aliasing thresholds and disturbed flows are aspects described in the following with respect to Fig. 7.

With optical flow processing, it is possible to obtain at least two velocity components. It is then more satisfactory to code flow direction parallel to the vessel axis rather than parallel to a hypothetical ultrasound beam. This vessel direction may vary in the image and the projection direction varies with it. As an example, red colour may be chosen for the most likely flow direction and blue colour for the opposite direction corresponding to reverse flows. The brightness of the colour represents the amplitude of the velocity component parallel to the vessel axis.

Further, artificial aliasing patterns are produced, using a velocity threshold $V_{th}$. This threshold may correspond to normal maximum velocity, in order to produce this aliasing phenomenon if velocity values are abnormally high. The aliased velocity $\tilde{V}$ is computed through a modulo operation in the following way:

$$V = kV_{th} + \tilde{V} \quad , \quad k \in \mathbb{Z}$$

$$\left|\tilde{V}\right| \leq V_{th}\Big/2 \, .$$

The aspect of disturbed flows comprises velocity variance, transverse velocity corresponding to projection perpendicular to the vessel axis and vorticity. According to the invention these aspects are envisaged to be colour coded as well. Figure 7 presents the operations corresponding to these imaging options.

In a vessel geometry determination 122 a vessel direction map $\vec{D}$ necessary for flow projection vector is computed using the vessel reference image $A(t_r)$. After vessel segmentation, the vessel orientation is computed using standard oriented ridge filters, and extended on the vessel map, creating the vessel direction map $\vec{D}$. This operation is performed one time for the complete sequence.

Further, longitudinal $V_{longitudinal}(t)$ and transverse $V_{transverse}(t)$ components of the velocity field are computed in a velocity projection step 124. $V_{longitudinal}(t)$ is colour coded using the aliasing threshold $V_{th}$.

Moreover, velocity variance, i.e. flow variance, is computed in a step 126. Vorticity is computed in a step 128. Transverse velocity variance, and vorticity are classically colour coded with dedicated colour maps in respective steps 130, 132, 134 and 136.

Finally an image composition 138 consists in the superimposition of the colour flow image over the grey X-ray image $I_{ca}(t_r)$ frozen at time $t_r$. This superimposed image tends to mimic colour flow imaging sequences produced by ultrasound modality but shows additional information by the means of the X-ray image as well.

As the invention provides the creation of a dynamic colour imaging sequence showing blood velocity values or blood flow characteristics within arteries, it shall be noted, that it is not necessary to emulate every characteristic of Ultrasound Colour Flow Imaging, but at least flow direction and its colour coding should be taken into account.

Among other aspects, the colour flow imaging according to the invention offers cardiologists and neurologists a way of evaluating vascular lesions, allowing to achieve interventions with better control on quality of resulting blood flow. While the invention has been illustrated and described in details in the drawings and forgoing description, such illustration and description are to be considered illustrative or exemplary and not restrictive; the invention is not limited to the disclosed embodiments.

**Claims**

1. An examination apparatus for representing blood flow-related information, the examination apparatus comprising:

   - at least one X-ray image acquisition device (140); and
   - a data processing unit (148);

   wherein the X-ray image acquisition device is arranged to generate a first image sequence and to generate a second image sequence at a contrast phase, whereby in the second image sequence a part of the subject has a different contrast than in said first image sequence;
   wherein the data processing unit (148) is arranged to determine a corresponding image of the first sequence to each image of the second sequence, using a time registration process; to subtract the first corresponding image from the second corresponding image to generate a DSA image; **characterized in that** said data processing unit is further arranged to register the vessel including a definition of a reference vessel image at a reference time after DSA through the detection of sufficient contrast or by the user; to register between the reference vessel image and a current vessel image; and to warp the current vessel image towards the reference vessel image generating an output registered contrast image; and to time filter the output sequence to extract time contrast modulation (16); and to compute vector velocity fields (18) applying motion estimation processing on the time filtered sequence; and to transform the velocity data into sound data generating a sound signal (20); and **characterized in that** the apparatus comprises at least one sound device (152) arranged to provide the sound signal (20).

2. The apparatus according to claim 1, comprising a display (150); wherein the data processing unit is arranged to transform the velocity data into a colour flow image; and
   wherein the display is arranged for displaying the registered contrast X-ray image and the colour flow image superimposed to the X-ray image.

3. X-ray imaging system with an apparatus according to one of the proceeding claims.


**Patentansprüche**

1. Untersuchungsgerät zur Darstellung von blutströmungsbezogenen Informationen, wobei das Untersuchungsgerät Folgendes umfasst:

   - mindestens eine Röntgenbildaufnahmevorrichtung (140); und
   - eine Datenverarbeitungseinheit (148);

   wobei die Röntgenbildaufnahmevorrichtung vorgesehen ist, um eine erste Bildsequenz zu erzeugen und um eine zweite Bildsequenz bei einer Kontrastphase zu erzeugen, wobei ein Teil des Patienten in der zweiten Bildsequenz einen anderen Kontrast hat als in der genannten ersten Bildsequenz;
   wobei die Datenverarbeitungseinheit (148) vorgesehen ist, um unter Anwendung eines Zeitregistrierungsprozesses ein entsprechendes Bild der ersten Sequenz für jedes Bild der zweiten Sequenz zu ermitteln; um das erste entsprechende Bild von dem zweiten entsprechenden Bild zu subtrahieren, um ein DSA-Bild zu erzeugen; **dadurch gekennzeichnet, dass** die genannte Datenverarbeitungseinheit weiterhin vorgesehen ist, um das Gefäß zu registrieren, einschließlich einer Definition eines Referenzgefäßbildes zu einem Referenzzeitpunkt nach der DSA über die Detektion von ausreichendem Kontrast oder durch den Benutzer; um eine Registrierung zwischen dem Referenzgefäßbild und einem aktuellen Gefäßbild durchzuführen; und um das aktuelle Gefäßbild zum Referenzgefäßbild hin zu verzerren und dadurch ein ausgegebenes registriertes Kontrastbild zu erzeugen; und um die ausgegebene Sequenz einer Zeitfilterung zu unterziehen, um die Zeitkontrastmodulation (16) zu extrahieren; und um Vektorgeschwindigkeitsfelder (18) zu berechnen, wobei die Bewegungsschätzungsverarbeitung auf die zeitgefilterte Sequenz angewendet wird; und um die Geschwindigkeitsdaten in Schalldaten umzuwandeln und somit ein Schallsignal (20) zu erzeugen; und **dadurch gekennzeichnet, dass** das Gerät mindestens eine Schallvorrichtung (152) umfasst, die vorgesehen ist, um das Schallsignal (20) zu liefern.

2. Gerät nach Anspruch 1, mit einer Anzeigevorrichtung (150);
   wobei die Datenverarbeitungsvorrichtung vorgesehen ist, um die Geschwindigkeitsdaten in ein farbiges Strömungsbild umzuwandeln; und
   wobei die Anzeigevorrichtung vorgesehen ist, um das registrierte Kontraströntgenbild und das farbige Strömungsbild dem Röntgenbild überlagert anzuzeigen.

3. Röntgenbildgebungssystem mit einem Gerät nach einem der vorhergehenden Ansprüche.

## Revendications

1. Appareil d'examen pour représenter des informations connexes au flux sanguin, l'appareil d'examen comprenant :

   - au moins un dispositif d'acquisition d'image radiologique (140) ; et
   - une unité de traitement de données (148) ;

   dans lequel le dispositif d'acquisition d'image radiologique est agencé pour générer une première séquence d'images et pour générer une seconde séquence d'images à une phase de contraste, moyennant quoi, dans la seconde séquence d'images, une partie de l'objet possède un contraste différent de celui dans ladite première séquence d'images ;
   dans lequel l'unité de traitement de données (148) est agencée pour déterminer une image correspondante de la première séquence à chaque image de la seconde séquence, en utilisant un procédé de mise en coïncidence temporelle ; pour soustraire la première image correspondante de la seconde image correspondante pour générer une image DSA ; **caractérisé en ce que** ladite unité de traitement de données est en outre agencée pour mettre le vaisseau en coïncidence, y compris une définition d'une image de vaisseau de référence, à un temps de référence après la DSA par l'intermédiaire de la détection de contraste suffisant ou par l'utilisateur ; pour réaliser une coïncidence entre l'image de vaisseau de référence et une image de vaisseau actuelle ; et pour déformer l'image de vaisseau actuelle vers l'image de vaisseau de référence, générant une image de contraste de sortie mise en coïncidence ; et pour filtrer en temps la séquence de sortie pour extraire une modulation de contraste temporelle (16) ; et pour calculer des champs de vitesses vectoriels (18) appliquant un traitement d'estimation de mouvement sur la séquence filtrée en temps ; et pour transformer les données de vitesse en données sonores, générant un signal sonore (20) ; et **caractérisé en ce que** l'appareil comprend au moins un dispositif sonore (152) agencé pour fournir le signal sonore (20).

2. Appareil selon la revendication 1, comprenant un affichage (150) ;
   dans lequel l'unité de traitement de données est agencée pour transformer les données de vitesse en une image de flux couleur ; et
   dans lequel l'affichage est agencé pour afficher l'image radiologique de contraste mise en coïncidence et l'image de flux couleur superposée sur l'image radiologique.

3. Système d'imagerie à rayons X avec un appareil selon une des revendications précédentes.

Fig. 1

EP 2 378 971 B1

Fig. 2

Fig. 3

Fig. 4

Fig. 5

Fig. 6

Fig. 7

Fig. 8

Fig. 9

EP 2 378 971 B1

**EP 2 378 971 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008107836 A
- US 4729379 A